# EUROPEAN PATENT APPLICATION

(11) **EP 1 342 779 A1**
(43) Date of publication of application: **10.09.2003**
(21) Application number: 02075874.4
(22) Date of filing: 06.03.2002
(51) Int. Cl.: C12N 5/06, C12N 5/08, C07K 14/705, C12N 15/12, A61K 38/17, G01N 33/50, A61P 37/00

(54) **Means and methods for manipulating hypersensitivity-like responses**

(71) Applicant: Fornix Biosciences N.V., 8243 PM Lelystad (NL)
(72) Inventor: Nijkamp, Franciscus Petrus, 3991 XM Houten (NL); Kraneveld, Aletta Desire-, 1393 PJ Nigtevegt (NL); Redegeld, Franciscus Antonius Maria, 3707 XM Zeist (NL)
(74) Representative: Prins, Adrianus Willem

(57) **Abstract**

Immunoglobulin light chains (lg-LC) are produced in excess in animals compared to heavy chains. The present invention implicates these Ig-LC in hypersensitivity responses and provides means and methods for manipulating the responses. The invention further provides a common gamma chain independent receptor on mast cells capable of mediating the mentioned effects of Ig-LC. In response to activation of the pathway of which the found receptor is a part, a mast cell is activated and stimulated to degranulate.

## Description

The invention relates to the field of medicine. The invention further relates to the fields of immunology and molecular biology. The invention in particular relates to means and methods for manipulating hypersensitivity responses.

Immunoglobulins (Ig) are important effector molecules of adaptive humoral immune responses. Production of IgE and IgG1 antibodies to innocuous antigens is a reflection of a normal immune response. Binding to Fc receptors, FcεRI or FcγRIII, on mast cells and basophiles, respectively and subsequent cross-linking of these Fc receptors can trigger hypersensitivity reactions. In patients with allergic syndromes, IgE is thought to play a central role in eliciting immediate hypersensitivity reactions. However, many hypersensitivity diseases do not always correspond with increased serum IgE levels or skin reactivity to common allergens. Studies in epsilon heavy chain gene-targeted mice (IgE-deficient) showed that active anaphylaxis can be induced in absence of IgE antibodies and was likely due to IgG1. On the other hand, immediate-like hypersensitivity reactions can also be elicited by other antigen-specific factors (1).

Tetrameric Ig's are produced and secreted by plasma cells. However, it is well documented that plasma cells also produce and secrete single Ig light chains (Ig-LC) in excess over Ig heavy chains or gamma chains (2-6). In vivo turnover studies in humans demonstrated that apparently only 60% of synthesized Ig-LC was incorporated into isotypic whole Ig and the remaining fraction was released into serum as free Ig light chain (7). Therefore, Ig-LC can be detected at low levels in normal serum and also in urine and in cerebrospinal fluid. Single Ig light chain and Ig light chain dimers bind antigen specifically. Antigen-binding affinities vary and are generally lower, but also affinities similar to or higher than tetrameric Ig were reported (8-15). It is against current dogma that secreted Ig-LC may play a physiological role, although in some instances free Ig-LC were found to display antigen-specific proteolytic activity (16, 17). However, in this study we show that Ig-LC can transfer immediate hypersensitivity-like responses in mice. These hypersensitivity-like responses are mast cell-mediated and are not detectable in mast cell-deficient mice. Shortly after antigen exposure of Ig-LC -sensitized animals, mast cell activation and local tissue swelling can be detected. We here show that Ig-LC can serve as a link in the mechanism by which mast cells regulate a number of immune-mediated diseases.

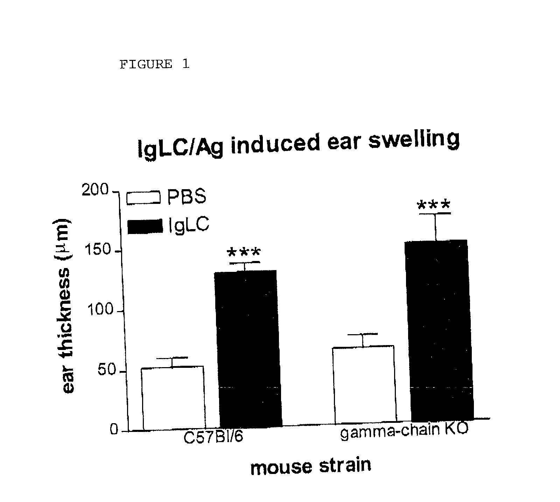

Mast cells are important for the development of acute responses in allergic reactions. Thus far, triggering the high affinity IgE receptor (FcεRI) and the low affinity IgG-receptor (FcγRIII) are the only routes known to activate mast cells in an antigen-specific manner. The present invention discloses that Ig-LC can exert their action independent from activation of the FcγRIII or FcεRI receptors. Both receptors signal via the common γ-chain and can trigger hypersensitivity reactions via activation of mast cells. Passive sensitization of animals deficient in the common γ-chain (FcRγ-/-) resulted in similar ear swelling responses after hapten challenge as compared to wild type (C57BL/6) animals (figure 1). This result shows that Ig light chains interact with a receptor which does not need the common γ-chain for signaling and thereby excludes FcεRI and FcγRIII or other gamma-chain associated receptors as effector molecules in Ig-LC -induced hypersensitivity .

In one aspect, the present invention thus provides an isolated cell comprising an Ig-LC receptor capable of activating a signal transduction pathway in said cell upon binding and crosslinking of an Ig-LC to said receptor, wherein said signal transduction is independent of the presence of a functional gamma-chain on said cell. Said isolated cell preferably comprises a mast cell, or a gamma chain-associated receptor-deficient cell, or mast cell

Knowledge of the presence of gamma chain independent receptor for Ig-LC opens the route to the identification of the receptor. Many techniques are present in the art to find receptors for ligands. The invention preferably utilizes protein solutions that are enriched for Ig-LC receptors. In one embodiment the invention therefore provides a method for obtaining a protein solution enriched for a receptor for Ig-LC comprising providing a gamma chain deficient mast cell with Ig-LC and cross-linking said Ig-LC to proteins in its vicinity and purifying Ig-LC and linked proteins from non-linked proteins. This purification can be achieved through many different means. In a preferred embodiment this is achieved through magnetic beads associated with said Ig-LC. Magnetic beads are coupled to Ig-LC. These beads are then incubated with mast cells or mast cell proteins and after washing the cells, Ig-LC (bait) are chemically cross-linked to cell surface proteins in immediate proximity to the binding site. After lysing the cells, Ig-LC -cross-linked cell surface proteins are separated from non-bound proteins using a magnetic device. Proteins are washed and separated using SDS-PAGE. This can be done using gel electrophoresis methods . Proteins can be characterized and identified by sequencing (part of) the proteins from certain positions in the gel. Proteins are further characterized and identified with Maldi-TOF mass spectrometry and/or Edman degradation. Binding of Ig-LC conjugated magnetic beads coupled to mast cells was visualized in the present invention by light microscopy (see examples). Binding was specific for light chain, since no binding was detected when beads were conjugated to bovine serum albumin (BSA). The process mentioned above is of course very much facilitated by the availability of the results of the genome project. Even very limited information of the sequence can currently be used to at least limit the number of candidate molecules. In many cases even limited information on the sequence is sufficient to identify a single candidate molecule responsible for the binding to mast cells. Depending on the identified candidates, different methods can be utilized to prove that molecule is the receptor.

Thus in another aspect the invention provides a purified and/or isolated and/or recombinant Ig-LC receptor or a functional part, derivative and/or analogue thereof, capable of activating an Ig-LC-dependent signal transduction pathway in a cell, wherein said activation is for example, but not limited to, an ion channel, said activation being independent of the presence of a functional gamma-chain receptor on said cell. A functional part of a Ig-LC receptor is a part comprising the same Ig-LC binding capabilities. A derivative of the mentioned receptor or part, can be obtained through (conservative) amino-acid substitution. As a functional part, or an analogue also a derivative comprises the same Ig-LC binding capabilities in kind, not necessarily in amount. Once a protein is identified, it is within the power of a person skilled in the art to provide a nucleic acid encoding said protein. Thus the present invention also provides a nucleic acid encoding an Ig-LC of the invention receptor or a functional part, derivative and/or analogue thereof, capable of activating an Ig-LC dependent signal transduction pathway in a cell. Expression vectors can be made and these can be introduced into target cells. Thus these vectors are also part of the invention. For more efficient delivery such vectors may be packaged into gene delivery vehicles such as for example virus or virus -like particles. Such virus or virus-like particles are therefore also part of the invention. The artisan is further capable to generate antibodies that are specific for said gamma-chain independent Ig-LC receptor.

With the knowledge of the gamma chain independent Ig-LC receptor it is possible to find compounds that at least in part inhibit the signal transduction pathway of the Ig-LC receptor, an example of such a compound is a molecule that competes with the binding of Ig-LC. This can for instance be done through molecular modeling or by high-throughput screening. Moreover, knowledge that Ig-LC is capable of binding to the receptor can be used to find competing molecules. Thus the invention further provides a compound capable of at least in part inhibiting a signal transduction pathway of an Ig-LC receptor of the invention. Preferably, said compound comprises a molecule capable of competing with Ig-LC for binding to a gamma-chain-independent Ig-LC receptor. Preferably, said compound comprises an antagonist capable of competing with Ig-LC for binding to a gamma-chain-independent Ig-LC receptor. Competing molecules can be tested for their capacity to antagonize the action of an Ig-LC. Preferably, this testing comprises the degranulation of mast cells or an Ig-LC dependent signal transduction pathway in a cell, more preferably independent of the presence of a functional immunoglobulin or gamma chain-associated receptor on said cell.
In another embodiment, the invention provides a method for determining whether a compound is capable of at least in part inhibiting signal transduction of a gamma chain independent Ig-LC receptor comprising providing a gamma-chain-receptor deficient cell comprising said receptor with said compound and determining whether Ig-LC mediated signal transduction is at least in part inhibited in said cell. A suitable antagonist of Ig-LC is an Ig-LC mutated in the receptor binding site such that it is not able to activate the receptor any more. Thus the invention provides a compound capable of interacting with an Ig-LC receptor, capable of preventing sensitizing a mast cell. Preferably, said compound comprises an Ig-LC selected for its capacity not to elicit a signal transduction signal.

An antagonist can be used to prevent or reduce sensitizing a mast cell. Thus the invention also provides a method for preventing or reducing sensitizing a mast cell comprising providing said mast cell with an Ig-LC antagonist. Preferably, said antagonist comprises a substance capable of binding to Ig-LC receptor, and incapable of binding an antigen or incapable of activating the receptor after binding an antigen.

Now that the receptor is found, it is also possible to manipulate the signal transduction pathway the receptor is part of. This can be done most easily on the level of the receptor itself. It is for instance possible to provide libraries of mutated receptors. These libraries can be used to find a mutant that is capable of activating a mast cell, independent of the presence of a bound Ig-LC/antigen complex. Providing activators or antagonists or even Ig-LC one can manipulate activation of a mast cell. Thus the invention also provides the use of an Ig-LC receptor to modulate a mast cell activated immune response.

Clinical uses are also within the invention. For instance an animal suffering or at risk of suffering from a hypersensitivity response can be administered a compound of the invention, thereby reducing the hypersensitivity response or reducing the chance and/or extent with which a hypersensitivity response will appear. In another embodiment the invention provides a method for reducing a hypersensitivity response in an animal comprising providing said animal with a molecule capable of preventing binding of an Ig-LC to an Ig-LC receptor. Said molecule can be a receptor antagonist of the invention. Said molecule can also be a molecule capable of binding to an Ig-LC thereby preventing binding of said bound Ig-LC to a gamma-chain independent receptor or binding of antigen by the receptor-bound Ig-LC. The latter molecule is for the present invention called an Ig-LC antagonist or ligand antagonist. The invention further provides an Ig-LC antagonist capable of preventing binding of an Ig-LC to a gamma chain dependent receptor on mast cells. In one embodiment, a compound capable of at least in part inhibiting an Ig-LC signal transduction pathway comprises THP or uromoduline, or a functional part, derivative and/or analogue thereof. In a preferred embodiment said compound comprises a peptide comprising an amino acid sequence (AHWSGHCCL) and/or a functional part, and/or derivative and/or analogue thereof.

For the present invention a human is also considered to be an animal. In a preferred embodiment said animal comprises a mammal. More preferably, said mammal comprises a human.

The invention further provides a use of a compound , a compound capable of at least in part inhibiting an Ig-LC signal transduction pathway, preferably an Ig-LC antagonist or a receptor antagonist, in the preparation of a medicament for the treatment of immediate and delayed hypersensitivity responses such as contact dermatitis, asthma, psoriasis, inflammatory bowel disease, rheumatoid arthritis, Sjögren, and systemic lupus erythematosus, and/or multiple sclerosis. Preferably, said medicament is formulated and packaged for parenteral and/or oral administration. Preferably, said compound comprises THP or uromoduline, or a functional part, derivative and/or analogue thereof. In a preferred embodiment said compound comprises a peptide comprising an amino acid sequence (AHWSGHCCL) and/or a functional part, and/or derivative and/or analogue thereof .
The mentioned routes of administration allow the formation of a depot from which new antagonist is recruited over time, thus allowing for a more prolonged effect of the medicament compared to an intra-venous administration. The invention further provides a method of treatment of an animal suffering from or at risk of suffering from immediate or delayed hypersensitivity-like responses such as asthma, psoriasis, inflammatory bowel disease, rheumatoid arthritis, Sjögren, and systemic lupus erythematosus, and/or multiple sclerosis. The method comprising administering said animal a medicament comprising a compound of the invention such as an Ig-LC antagonist or a gamma chain independent receptor antagonist and a suitable carrier. In a preferred embodiment said disease comprises Multiple Sclerosis.

Free Ig-LC are produced and secreted by B lymphocytes and considerable levels of Ig-LC are present in serum. The present invention demonstrates that Ig-LC confer hypersensitivity in naïve animals. Mice passively sensitized with trinitrophenol (TNP)- or oxazolone (Ox)-specific Ig-LC develop a cutaneous swelling response, have elevated plasma histamine and show morphologic signs of mast cell degranulation after challenge with relevant antigen. Induction of hypersensitivity is independent of presence of the common gamma chain, excluding a role for Fc- or other gamma-chain associated receptors. Hapten specific Ig-LC is produced within 24 hour after topical sensitization with low molecular weight compounds trinitrophenol chloride (2-chloro 1,3,5 trinitrobenzene; picryl chloride (PCl)), 2,4-dinitrofluorobenzene (DNFB) or oxazolone by spleen cells from sensitized mice. Although it is clear that IgE and IgG₁ are central to the induction of immediate hypersensitivity reactions, the present invention shows that Ig-LC are similarly able to transfer hypersensitivity to naïve animals. Free Ig-LC are an as yet unappreciated-factor in the humoral immune response to antigen exposure, and can upon cross-linking lead to mast cell-dependent immediate hypersensitivity-like reactions.

In one aspect the invention provides evidence for a novel role for free Ig-LC. We show that Ig-LC transfer hapten sensitivity into naive animals and subsequent antigen challenge elicits an immediate hypersensitivity-like response, which appears to be mast cell dependent.

Mast cells have been implicated in a wide variety of biological responses including immediate hypersensitivity reactions, bacterial sepsis and also in T-cell dependent reactions such as contact hypersensitivity reactions, experimental autoimmune encephalomyelitis (EAE), or non-allergic asthma. The diseases like contact sensitivity, EAE and non-allergic asthma can be induced by local delayed type hypersensitivity (DTH) reactions and are independent of IgE or IgG1. Studies in mast cell-deficient animals show that mast cells are crucial in orchestrating a full DTH response. Humoral factors released by B cells seem important in contact hypersensitivity reactions since hypersensitivity is impaired in B-cell deficient animals .In ongoing studies, we have found that TNP-specific Ig-LC are capable of rescuing impaired contact hypersensitivity reactions as a result of hapten application in B-cell deficient mice. In addition, passive sensitization with Ig-LC can give rise to a rapid and profound airway bronchoconstriction in mice after intra-airway antigen challenge, a reaction dependent on mast cell activation. Importantly, Ig-LC are involved in delayed type hypersensitivity reactions leading to bronchoconstriction, cellular influx in bronchoalveolar lavage, and airway hyper reactivity after active sensitization with low molecular weight compounds followed by intranasal challenge (manuscript in preparation). It is of interest that the secretion of Ig-LC is augmented under pathological conditions such as multiple sclerosis, Sjögren's disease, systemic lupus erythematosus, and other neurological disorders (18-21) For example, production of Ig-LC in patients with multiple sclerosis is greatly enhanced (20, 22). This production of Ig-LC is associated with recent antigenic stimulation and correlates with severity of the disease. In concord with our hypothesis, it has been demonstrated that mast cells play an important role in the pathogenesis of MS or EAE; substantially reduced disease symptoms are found in mast cell-deficient animals.

### Examples

### Example 1: Ig-LC do not activate gamma-chain associated receptors

From our studies it is clear that mast cells are crucial for the development of acute responses in skin and airways leading to ear swelling and acute bronchoconstriction, respectively. Thus far, triggering the high affinity IgE receptor (FcεRI ) and the low affinity IgG-receptor (FcγRIII) are the only routes known to activate mast cells in an antigen-specific manner. We investigated whether Ig-LC exerted their action via activation of the FcγRIII or FcεRI receptors. Both receptors signal via the common γchain and can trigger hypersensitivity reactions via activation of mast cells. Passive sensitization of animals deficient in the common gamma chain (FcRγ-/-) resulted in similar ear swelling responses after hapten challenge as compared to wild type (C57BL/6) animals (Fig 1). This indicates that Ig-LC interact with a receptor, which does not need the common γchain for signaling and thereby excludes FcεRI and FcγRIII but also other gamma-chain associated receptors such as PIR-A and-B as effector molecules in Ig-LC -induced hypersensitivity reactions. Preliminary experiments showed that Ig-LC bind to mast cells and recognize antigen in rosetting assays with hapten-conjugated red blood cells. FACS analysis indicated that both IgE and IgG1 do not compete with the binding of Ig-LC to murine bone marrow-derived mast cells, confirming our results in the FcRγ-/- mice. Various attempts to directly activate murine bone marrow-derived mast cells in vitro to release prestored mediators after cross linking of surface-bound Ig-LC were not successful. Although in some experiments a significant degree of degranulation was found, results were highly variable. The latter may be explained by a possible lack of co stimulatory factor in vitro, a maturation-dependent expression of cell surface proteins involved in Ig-LC -mediated activation or the simultaneous presence of inhibitory and activatory receptors.

### Example 2: Biochemical isolation and purification of Ig-LC receptor.

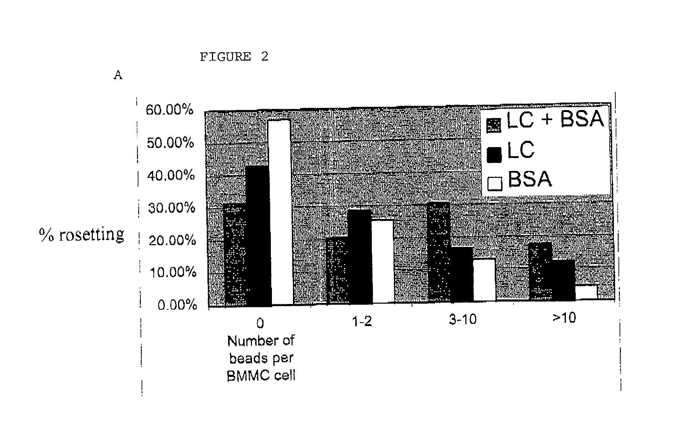

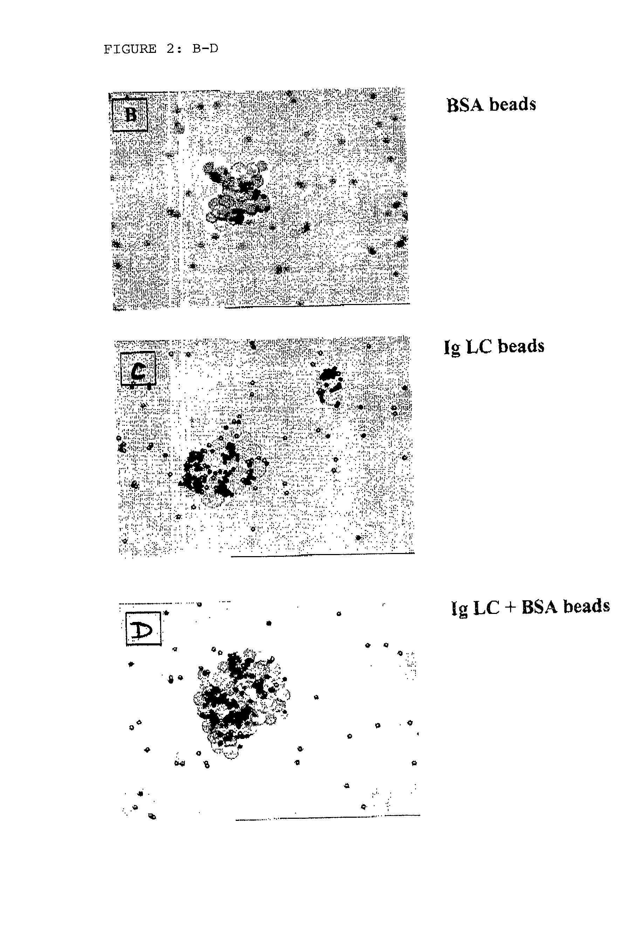

Chemical crosslinking of ligand to cellular membrane was the method employed to isolate and identify cell surface proteins as putative receptors for various ligands. Magnetic beads were coupled to Ig-LC. These beads were then incubated with murine bone marrow-derived mast cells and after washing the cells, Ig-LC (bait) were chemically cross linked to cell surface proteins in immediate proximity to the binding site. After lysing the cells, Ig-LC -cross linked cell surface proteins were separated from non-bound proteins using a magnetic device. Proteins were washed and separated using SDS-PAGE (1-D or 2-D). Proteins were further characterized and identified with Maldi-TOF mass spectrometry and/or Edman degradation. Binding of Ig-LC conjugated magnetic beads coupled to mast cells were visualized by light microscopy. Binding was specific for light chain, since no binding was detected when beads were conjugated to bovine serum albumin (BSA) (Fig 2 a-d).

### Example 3. Expression cloning of the Ig-LC receptor

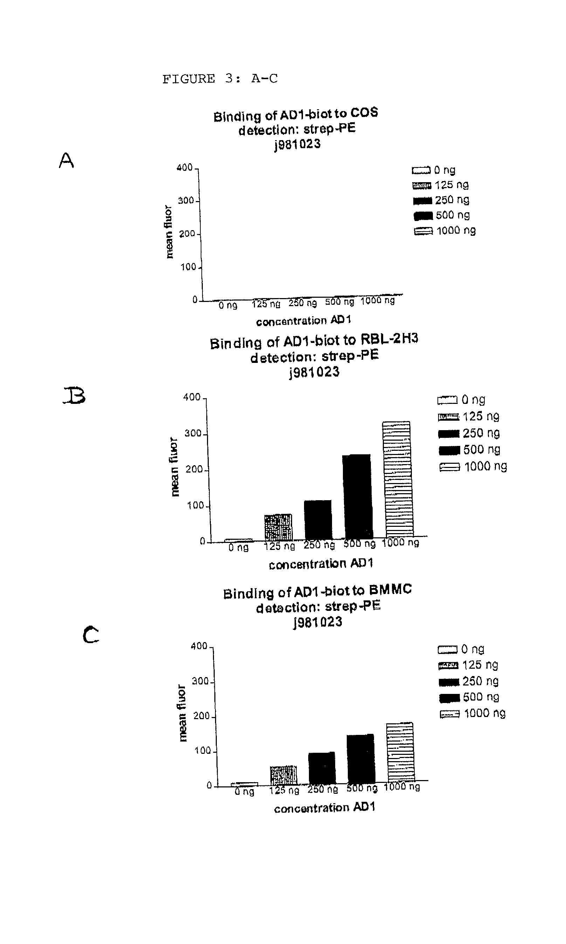

Expression cloning was used to clone an Ig-LC receptor. A cDNA library from primary cultured murine mast cells (BMMC) was constructed. This cDNA was transfected into mammalian cells. Transfected cells were compared in their binding capacity for Ig-LC using a flow cytometer. Cells binding Ig-LC above background were collected by the FACS sorter. Transfected DNA from these cells were isolated and used for succeeding transfection rounds. After several transfection/sorting rounds, a single Ig-LC receptor-expressing cell population was isolated. The transfected DNA from this population encodes for the putative Ig-LC receptor. A receptor present on mast cells of specific interest for binding Ig-LC is CD63, a transmembrane-5 (TM5) membrane protein This receptor is expressed by mast cells and cross-linking of this receptor results in mast cell activation and mediator release. FACS experiments showed the presence of CD63 on mast cell line RBL-2H3, but not on COS cells (figure 3).

### Example 4. Role of mast cells in Ig-LC responses

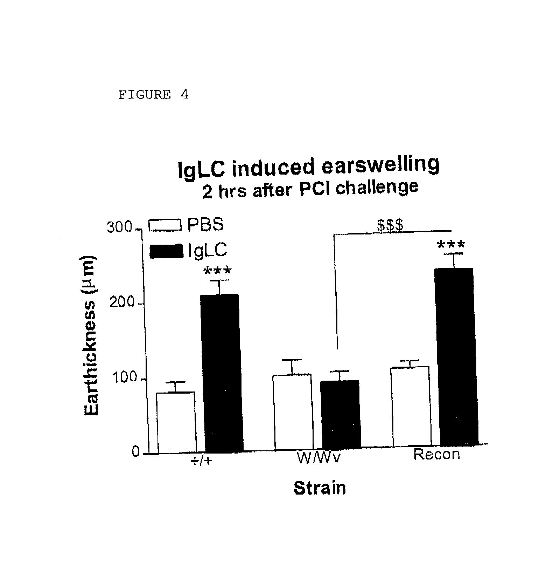

The involvement of mast cells in eliciting ear swelling responses after topical challenge of Ig-LC -sensitized mice was investigated using mast cell-deficient mice (W/Wv). As shown in figure 4, passive sensitization of mast cell-deficient mice with TNP-specific Ig-LC followed by topical application of TNP did not result in a significant ear swelling at 2 hours after challenge. As expected, similar treated control mast cell-sufficient littermates (+/+) showed normal ear swelling responses. To further proof that the absence of mast cells alone was responsible for the completely reduced ear swelling response in the mast cell-deficient mice, we reconstituted these animals with bone marrow-derived mast cells from wild-type animals. Reestablishment of the mast cell population by local injection of bone marrow-derived mast cells restored the ear swelling responses in Ig-LC -sensitized and hapten challenged animals (figure 4).

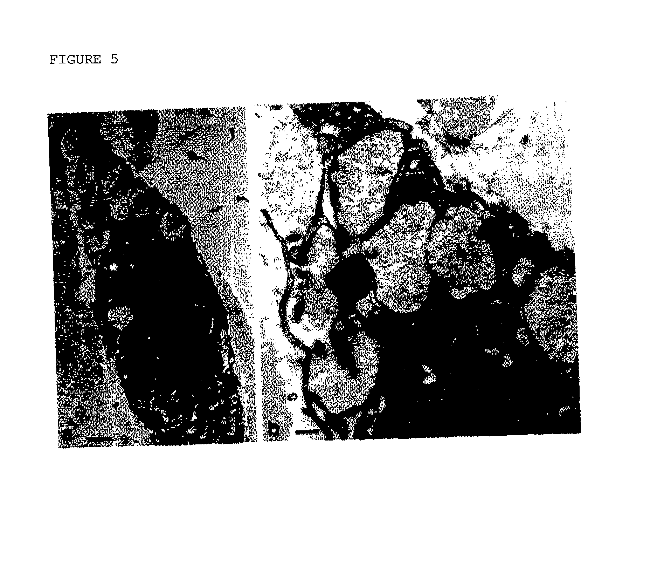

Indeed, hapten challenge of Ig-LC-sensitized animals was accompanied with a rapid increase in plasma histamine levels in vivo and direct proof for mast cell activation was obtained after histological and ultrastructural analysis of biopsies of the ears at 1 hour after topical hapten challenge. Mast cells in tissue sections of mice intravenously sensitized with TNP-specific Ig-LC showed marked signs of degranulation after re-exposure to hapten. Electron microscopy revealed that degranulation was characterized by swelling of intracytoplasmic granules, decrease of electron-density of the granules and extrusion of membrane-free granules from the mast cells (figure 5).

### Example 5. Specific recognition of antigen by Ig-LC.

In general, it is acknowledged that both heavy chain and light chain contribute to binding of antigens by immunoglobulins, which are monomers or multimers of a tetrameric structure consisting of two light chains and two heavy chains. The structure of an Ig-LC can be separated in a constant and variable region. The latter region contains hypervariable domains (CDR's), which are responsible for antigen recognition. The genes encoding these regions undergo rearrangement/mutation to effect affinity maturation and gain specificity for a certain antigen. Similar mechanisms play a role in shaping the antigen recognition by Ig heavy chains.

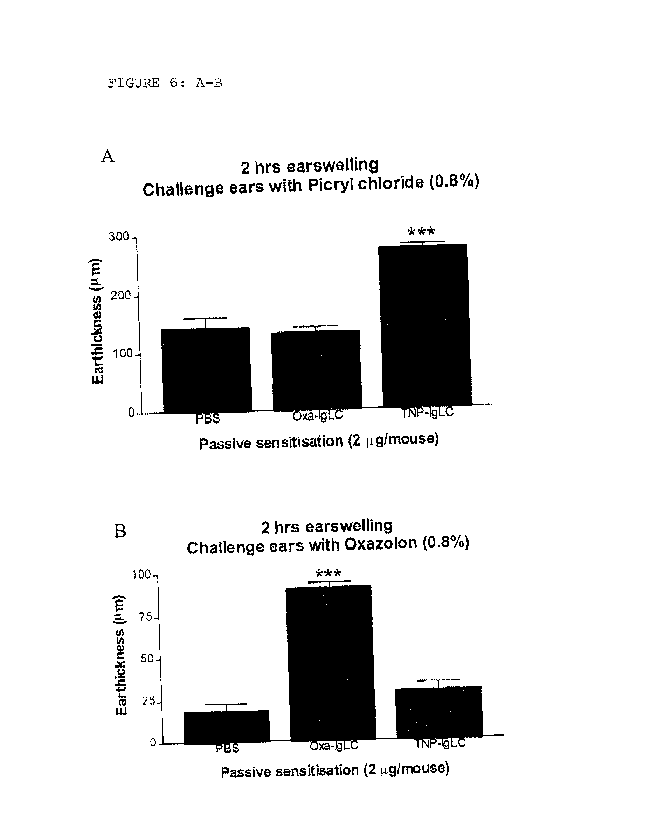

In order to prove that free Ig-LC have the capability to recognize and bind to antigen, we have performed the following experiments. First two Ig-LC's with different antigen specificity were generated by separation of immunoglobulin heavy and light chains from oxazolone- and TNP-specific IgG's. Naïve mice were sensitized with the isolated Ig-LC and subsequently topically challenged with TNP or oxazolone. Two hours after challenge ear thickness was measured. As shown in figure 6, when mice were sensitized with oxazolone-specific Ig-LC only an increase in ear thickness was measured when the ears were challenged with oxazolone and not after application of picryl chloride, i.e. TNP . Vice versa, when animals were sensitized with TNP-specific Ig-LC ear swelling was only induced by TNP and not by oxazolone.

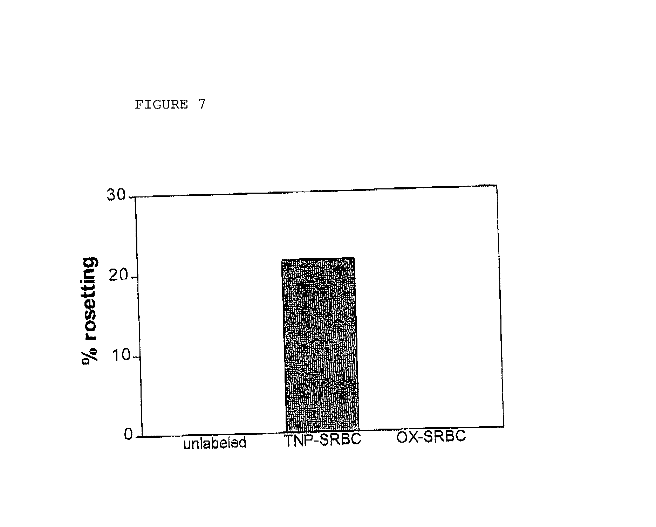

In a second experiment, we investigated if Ig-LC when bound to mast cells is able to recognize antigen in a proper way. In in vitro experiments, bone marrow-derived mast cells were sensitized with TNP-specific Ig-LC. Next, sensitized cells were co incubated with either unlabeled, TNP-labeled or oxazolone-labeled sheep red blood cells (SRBC). Binding (rosetting) of the SRBC was scored under a light microscope. As evidenced in figure 7, TNP-specific Ig-LC -sensitized mast cells only show significant binding to TNP-labeled SRBC, but not to unlabeled or with an unrelated hapten (OX)-coupled SRBC. This experiment confirms that Ig-LC are indeed able to specifically recognize antigen.

### Example 6: Production of Ig-LC after contact sensitization of mice.

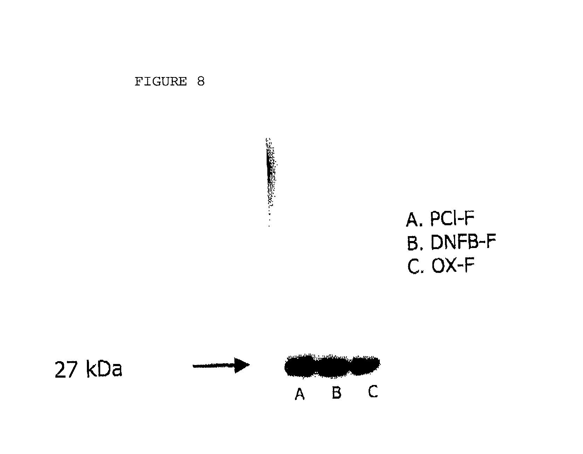

Induction of immediate hypersensitivity by Ig-LC s may be physiologically relevant. Ig-LC are produced and secreted upon antigen exposure. To test this hypothesis, spleen and lymph node cells from mice that had been sensitized epicutaneously to PCl, DNFB or oxazolone 4 days before, were cultured in vitro for 1 day in the absence of hapten. Hapten-binding proteins from culture supernatant were isolated by hapten-affinity chromatography. Western analysis showed that the hapten-binding factors obtained this way contained Ig kappa light chain (figure 8), but no Ig heavy chains were detected (data not shown). Importantly, TNP-binding factor could be isolated from culture supernatant of spleen and lymph node cells isolated from mice as early as 1 day after topical sensitization with PCl. In this preparation the presence of Ig-LC was confirmed by Western blot analysis (data not shown). The N-terminal amino acid sequence of this protein showed almost complete homology with mouse Ig kappa light chain.

### Example 7: Measurement Ig-LC in human serum.

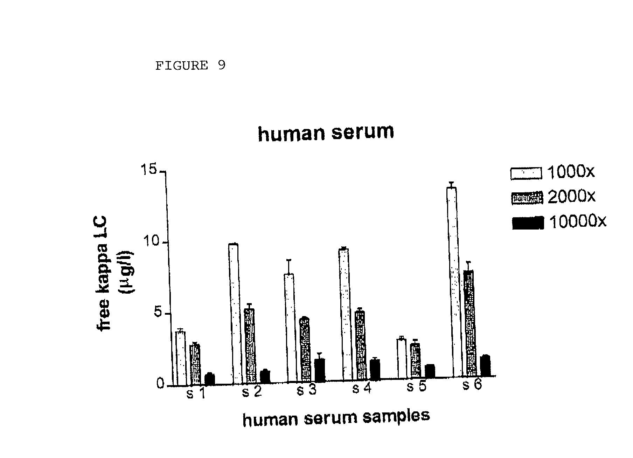

Free Ig-LC can be detected in various human body fluids e.g. liquor, urine and serum. Using an Ig-LC -specific ELISA we were able to detect significant levels of Ig-LC in serum (figure 9). The detected levels are comparable to these reported earlier by other groups.

### Example 8: Effects of F991, an Ig-LC binding peptide.

F991 is developed as an antagonist of Ig light chain. The working hypothesis for this compound is that it binds to Ig light chains and thereby prevents binding of light chains to their putative receptors. F991 is a 9-mer peptide derived from the endogenous protein uromodulin. The potency of F991 to inhibit Ig-LC induced cutaneous reactions (ear swelling responses) was studied. Our working hypothesis for this compound is that it is an antagonist of Ig-LC and binds to Ig-LC and thereby prevents binding of Ig-LC to their putative receptors. F991 was administered in different dosages, via various administration routes.

### A. Dose dependent inhibition after intravenous administration.

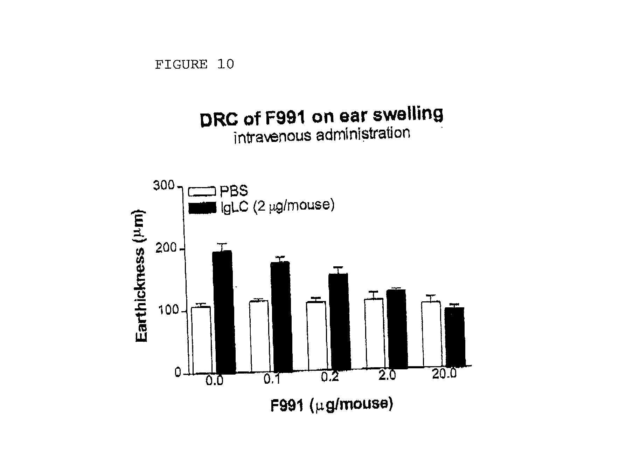

Indicated amounts of F991 per mouse were intravenously injected at 30 min before hapten challenge. At that same time point mice were injected (passively sensitized) with TNP-specific Ig-LC or vehicle (PBS). Two hours after hapten challenge increase in ear thickness was monitored. The figure 10 shows a clear dose-dependent inhibition of the ear swelling response by F991. Amounts of 2 µg per mouse (1.9 nmole!!) were sufficient to completely block the Ig-LC -induced ear swelling (Figure 10).

### B. Other administration routes: Intraperitoneal administration of F991

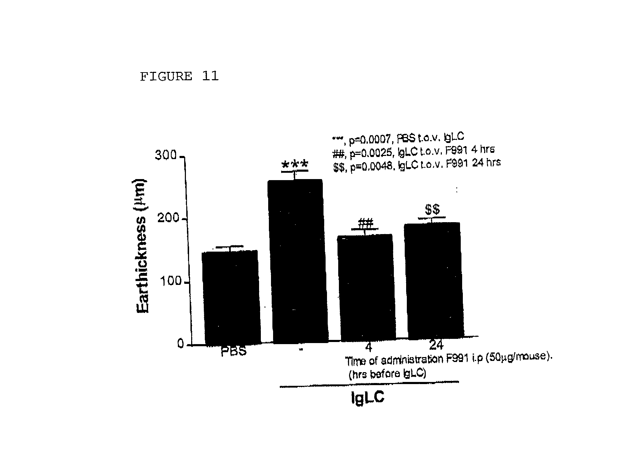

Mice were injected with 50 µg of F991 intraperitoneally at 4 or 24 hours before challenge with hapten. 30 min before challenge the animals were passively (i.v.) sensitized with TNP-specific Ig-LC and two after hapten application onto the ears, ear thickness was measured. As demonstrated in the figure 11, i.p. administration of F991 even at 24 hours before hapten challenge completely prevented the induction an ear swelling response (Figure 11).

### C. Subcutaneous administration of F991

Protocol see above for i.p. administration, except F991 was injected subcutaneously.

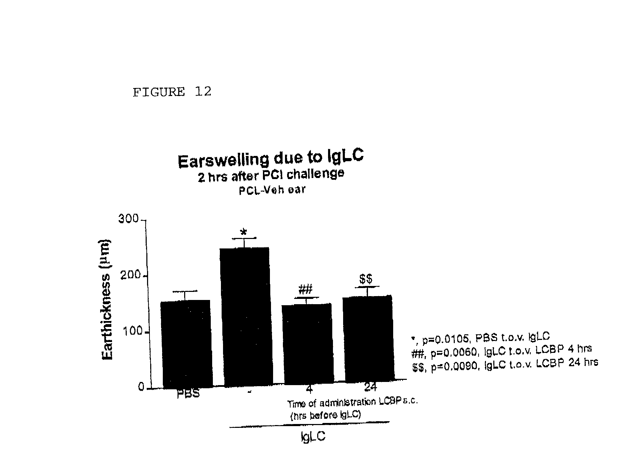

Similar to the i.p. administration of F991 when injected subcutaneously, F991 again completely inhibited Ig-LC -induced ear swelling after hapten challenge (Figure 12).

### Example 9. Epicutaneous application of F991 (in ointment)

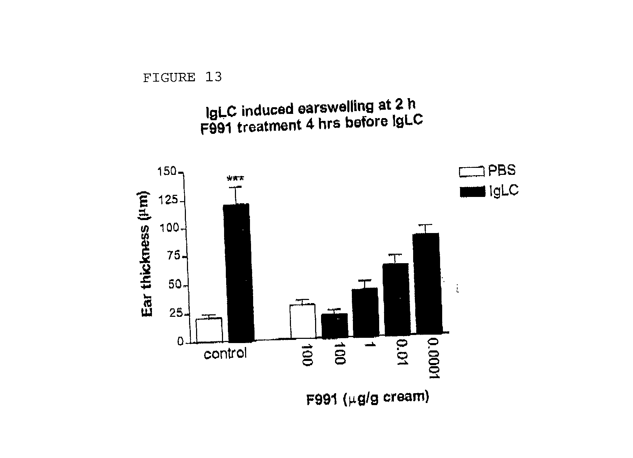

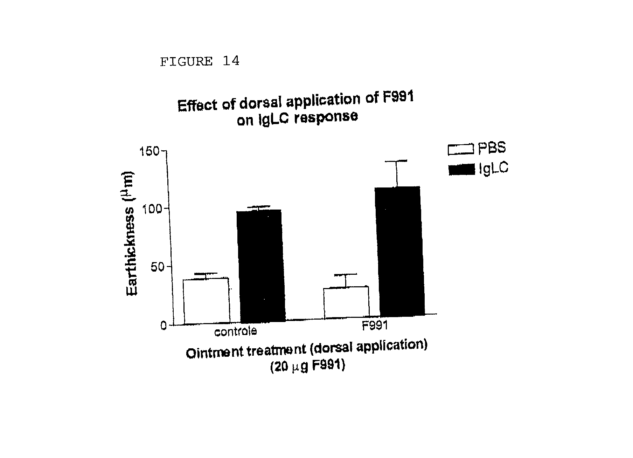

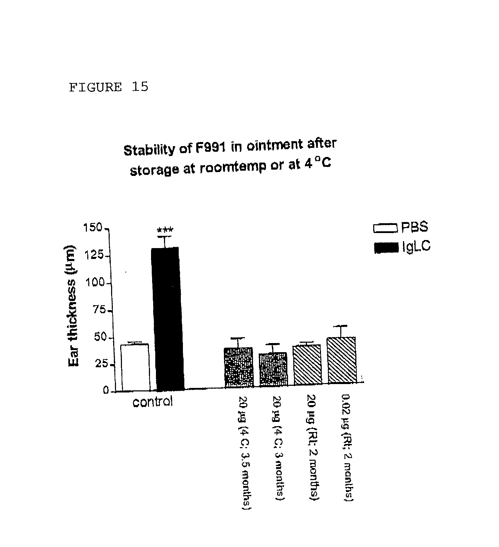

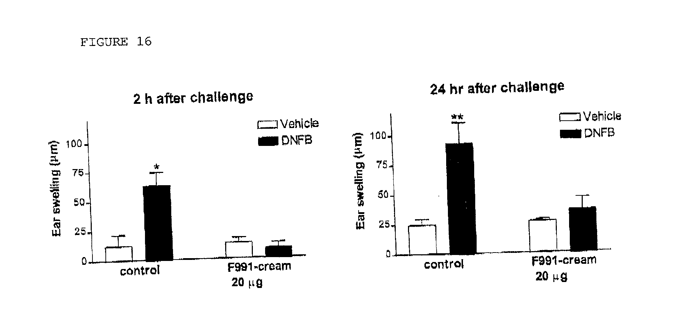

A. F991 was prepared as an ointment in Cremor Cetomacrogolis FNA and topically applied on the ear at 4 hours before hapten challenge. Subsequently, mice were passively sensitized with 2 µg TNP-specific Ig-LC n at 30 min before and ear thickness was measured 2 hours after hapten challenge of the ears. As shown in the figure 13, topical application of F991 in an ointment resulted in a dose-dependent inhibition of the ear swelling induced after hapten challenge of Ig-LC sensitized animals (Figure 13).
B. We further investigated if local application of F991 as an ointment resulted in systemic inhibition of the Ig-LC -induced effects. Therefore F991 was applied on the back of the mice instead of at the site of hapten challenge (ear). Mice were again sensitized and challenged as described above. The figure 14 shows that topical application of F991 does not result in systemic inhibition of the Ig-LC effects. This means that epicutaneous treatment should be done at the site of challenge (Figure 14).
C. Stability of F991 in ointment
   To determine the stability of F991 in ointment, different preparations of F991 in Cremor Cetamacrogolis FNA were tested for their activity after storage for 2-3 months at room temperature and at 4 degrees Celsius. The different preparations were applied at the ear as described in A. and subsequently ear swelling was monitored after hapten challenge of Ig-LC-sensitized animals. As shown in the figure 15, F991 stored under all different conditions retained its activity (Figure 15).
D. Next we investigated if topical treatment with F991 also inhibited the development of contact sensitivity reactions induced after active sensitization. Mice were sensitized with a low molecular weight compound DNFB on the skin and footpads (on day 0 and 1). 5 days after the start of sensitization and 4 hours before local challenge with hapten, mice were treated with F991 on the ears. Two hours and 24 hours after hapten challenge the increase in ear thickness was determined. As shown in the figure 16, topical treatment with F991 completely inhibited the ear swelling at both 2 and 24 hours after challenge. This experiment indicates that topical treatment with F991 may be of therapeutic use in the treatment of contact dermatitis, a disease with similar characteristics (Figure 16).

### Example 10: Ig-LC, mast cells and multiple sclerosis.

It is well documented that production of Ig-LC in patients with multiple sclerosis is greatly enhanced. Elevated levels of free Ig-LC can be detected in cerebrospinal fluid and urine of MS patients. This production of free Ig-LC is associated with recent antigenic stimulation and correlates with severity of the disease. In concord with our hypothesis, it has been demonstrated that mast cells play an important role in the pathogenesis of MS; substantially reduced disease symptoms are found in mast cell-deficient animals (23) Further, mast cells are observed in CNS plaques, and histamine and tryptase levels are elevated in the liquor of MS patients, whereas treatment with mast cell stabilizers or antagonists of histamine and serotonin seem to ameliorate MS.

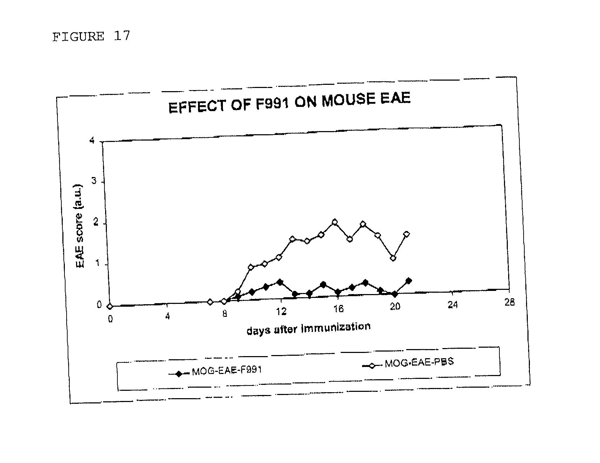

If light chains were involved in the activation of mast cells in patients with MS, the prediction is that F991 may be of therapeutic interest in the treatment of MS. We tested if F991 was able to prevent of reduce development clinical signs in an established mouse model for MS, i.e. myelin oligodendrocyte glycoprotein (MOG)-induced experimental allergic encephalomyelitis (EAE). Mouse were sensitized with antigenic peptide 35-55 from MOG in complete freund adjuvant. At the day of sensitization and 3 days later, mice also received an injection with *pertussis* toxin. In general, 10-12 days after the start of sensitization mice develop clinical signs of MS, which can be scored in degree of paralysis (0=no paralysis, 1=tail flaccidity, 2=hind limb weakness, 3=hind limb paralysis, 4=forelimb paralysis or loss of ability to right supine, 5=death). To investigate if F991 is able to prevent development of clinical signs of MS, mice were daily injected with 50 µg F991/animal i.p. starting at day -1 until day 21. As shown in the figure 17, treatment with F991 completely prevented the development of clinical signs of paralysis. Control mice developed disease with a mean day of onset of 11.8 and a mean clinical score of 2.3. There was a significant difference in disease burden (mean cumulative EAE score) between the F991 (mean 4.2) treated group and the control group (mean 18.3). Termination of the treatment with F991 at 21 days after sensitization resulted in the development of some minor clinical symptoms ("silly walk"), but not in a clear manifestation of EAE/MS (data not shown). (Figure 17).

### References

1. Van Loveren, H., R. E. Ratzlaff, K. Kato, R. Meade, T. A. Ferguson, G. M. Iverson, C. A. Janeway, and P. W. Askenase. Immune serum from mice contact-sensitized with picryl chloride contains an antigen-specific T cell factor that transfers immediate cutaneous reactivity. *Eur J Immunol* **16**(10):1203-8. (1986).
2. Hopper, J. E., and E. Papagiannes. Evidence by radioimmunoassay that mitogen-activated human blood mononuclear cells secrete significant amounts of light chain Ig unassociated with heavy chain. *Cell Immunol* **101**(1):122-31. (1986).
3. Shapiro, A. L., M. D. Scharff, J. V. Maizel, and J. W. Uhr. Synthesis of excess light chains of gamma globulin by rabbit lymph node cells. *Nature* **211**(46):243-5. (1966).
4. Skvortsov, V. T., and A. E. Gurvich. Relative rates of synthesis of immunoglobulins and light chains in rabbit spleen cells during secondary response. *Nature* **218**(139):377-8. (1968).
5. Hannam-Harris, A. C., J. Gordon, and J. L. Smith. Immunoglobulin synthesis by neoplastic B lymphocytes: free light chain synthesis as a marker of B cell differentiation. *J Immunol* **125**(5):2177-81. (1980).
6. Hannam-Harris, A. C., and J. L. Smith. Free immunoglobulin light chain synthesis by human foetal liver and cord blood lymphocytes. *Immunology* **43**(3):417-23. (1981).
7. Waldmann, T. A., W. Strober, and R. P. Mogielnicki. The renal handling of low molecular weight proteins. II. Disorders of serum protein catabolism in patients with tubular proteinuria, the nephrotic syndrome, or uremia. *J Clin Invest* **51**(8):2162-74. (1972).
8. Yoo, T. J., O. A. Roholt, and D. Pressman. Specific binding activity of isolated light chains of antibodies. *Science* **157**(789):707-9. (1967).
9. Masat, L., M. Wabl, and J. P. Johnson. A simpler sort of antibody. *Proc Natl Acad Sci U S A* **91**(3):893-6. (1994).
10. Sun, M., L. Li, Q. S. Gao, and S. Paul. Antigen recognition by an antibody light chain. *J Biol Chem* **269**(1):734-8. (1994).
11. Mahana, W., F. Jacquemart, and M. Ermonval. A murine monoclonal multireactive immunoglobulin kappa light chain. *Scand J Immunol* **39**(1):107-10. (1994).
12. Ledbetter, J. A., H. P. Fell, L. S. Grosmaire, N. A. Norris, and T. T. Tsu. An immunoglobulin light chain dimer with CD4 antigen specificity. *Mol Immunol* **24**(12):1255-61. (1987).
13. Schechter, I., and E. Ziv. Binding of 2,4-dinitrophenyl derivatives by the light chain dimer obtained from immunoglobulin A produced by MOPC-315 mouse myeloma. *Biochemistry* **15**(13):2785-90. (1976).
14. Painter, R. G., H. J. Sage, and C. Tanford. Contributions of heavy and light chains of rabbit immunoglobulin G to antibody activity. I. Binding studies on isolated heavy and light chains. *Biochemistry* **11**(8):1327-37. (1972).
15. Tribbick, G., A. B. Edmundson, T. J. Mason, and H. M. Geysen. Similar binding properties of peptide ligands for a human immunoglobulin and its light chain dimer. *Mol Immunol* **26**(7):625-35. (1989).
16. Thiagarajan, P., R. Dannenbring, K. Matsuura, A. Tramontano, G. Gololobov, and S. Paul. Monoclonal antibody light chain with prothrombinase activity. *Biochemistry* **39**(21):6459-65. (2000).
17. Sun, M., Q. S. Gao, L. Li, and S. Paul. Proteolytic activity of an antibody light chain. *J Immunol* **153**(11):5121-6. (1994).
18. Fagnart, O. C., C. J. Sindic, and C. Laterre. Free kappa and lambda light chain levels in the cerebrospinal fluid of patients with multiple sclerosis and other neurological diseases. *J Neuroimmunol* **19**(1-2):119-32. (1988).
19. Moutsopoulos, H. M., A. D. Steinberg, A. S. Fauci, H. C. Lane, and N. M. Papadopoulos. High incidence of free monoclonal lambda light chains in the sera of patients with Sjogren's syndrome. *J Immunol* **130**(6):2663-5. (1983).
20. Solling, K., J. Solling, and F. K. Romer. Free light chains of immunoglobulins in serum from patients with rheumatoid arthritis, sarcoidosis, chronic infections and pulmonary cancer. *Acta Med Scand* **209**(6):473-7 (1981).
21. Lamers, K. J., J. G. de Jong, P. J. Jongen, M. J. Kock-Jansen, M. A. Teunesen, and E. M. Prudon-Rosmulder. Cerebrospinal fluid free kappa light chains versus IgG findings in neurological disorders: qualitative and quantitative measurements. *J Neuroimmunol* **62**(1):19-25. (1995).
22. Mehta, P. D., S. D. Cook, R. A. Troiano, and P. K. Coyle. Increased free light chains in the urine from patients with multiple sclerosis. *Neurology* **41**(4):540-4. (1991).
23. Secor, V. H., W. E. Secor, C. A. Gutekunst, and M. A. Brown. Mast cells are essential for early onset and severe disease in a murine model of multiple sclerosis. *J Exp Med* **191**(5):813-22. (2000).

### Legends to the figures

### Figure 1.

Ear swelling induced by crosslinking of Ig light chains is not dependent on Fc-receptors. Common gamma chain knockouts and control mice (C57B1/6) were passively sensitized with TNP-specific Ig light chain or vehicle (PBS). Ears were challenged with picryl chloride (TNP) and ear thickness was measured 2 hours later.

### Figure 2.

a. Quantification of binding of magnetic beads derivatized with albumin (BSA), Ig light chain plus BSA (to improve orientation of Ig light chain). Binding was scored using light microscopy and number of beads attached per cell is shown.
b. Miscroscopic visualisation of binding Ig light chain-coupled magnetic beads to primary cultured murine bone marrow- derived mast cells (BMMC). BMMC incubated with beads coupled to BSA, showing minor degree of binding.
c. Ig light chain-derivatized magnetic beads showing clear rosetting of beads around intact cells.
d. Ig light chain-derivatized magnetic beads (Ig light chain + BSA to correct orientation of the light chains) showing clear rosetting of beads around intact cells.

### Figure 3.

Expression of CD63 on COS fibroblast-like cells (a), a rat mast cell line RBL-2H3 (b) and primary cultured murine mast cells BMMC (c). Expression is detected by FACS with an antibody AD1 specific for CD63.

### Figure 4.

Mast cell dependency of hapten-induced ear swelling in Ig LC sensitised mice. Mast cell-deficient (W/W^{v}) or congenic controls (+/+) were intravenously sensitised with TNP-sepcific Ig light chain (5 mg). 30 minutes after sensitisation mice were topically challenged with PCl and ear thickness was monitored at 2 hours after challenge. Ear swelling in W/W^{v} significantly different from +/+, p<0.05. Local reconstitution of the right ear with bone marrow-derived mast cells in W/W^{v} mice, 3 weeks prior to the experiment resulted in a complete recovery of the sensitivity to Ig light chain.

### Figure 5.

Electron micrograph of a mildly degranulated mast cell in the dermis of the ear of a TNP-specific Ig light chain sensitised mouse at 1 hour after topical challenge with hapten. Numerous granules remain unaltered, some secretory granules are enlarged, exhibit diminished electron density and release their content (enlargement in b).

### Figure 6.

Injection of TNP- or oxazolone-specific Ig light chain resulted in antigen-specific transfer of hapten sensitivity. Mice were intravenously injected with TNP-specific Ig light chain or oxazolone-specific Ig light chain and challenged on the ear with trinitrophenol chloride (PCl) (panel A) or oxazolone (Oxa) (panel B). Controls received vehicle (PBS) were also topically challenged with both haptens. Ear swelling was measured 2 h after challenge

### Figure 7.

Mast cells sensitised with Ig light chain recognize antigen in a specific manner. TNP-specific Ig light chain sensitized cultured mast cells (BMMC) were incubated with TNP- or oxazolone (OX)-conjugated SRBC; rosetting cells were scored using light microscopy.

### Figure 8.

Antigen-specific Ig light chain is produced after *in vivo* skin sensitisation with low molecular weight compounds. Immunoblotting of antigen-binding factors specific for 2-chloro-1,3,5-trinitrobenzene (A), dinitrofluorobenzene (B) and oxazolone (C) with Ig kappa light chain-specific antibody.

### Figure 9

Measurement of free kappa Ig light chains in human serum using an Ig kappa light chain-specific ELISA. Samples of 6 human subjects were analyzed at different dilutions (1000, 2000, and 10000 fold diluted).

### Figure 10

Intravenous administration of different amounts of F991 at 30 min before hapten challenge of Ig light chain-sensitized mice results in a dose-dependent inhibition of ear swelling after hapten challenge. Mice were i.v. sensitized with 2 µg TNP-specific Ig light chain at 30 min before ear challenge with TNP. Control mice were injected with PBS instead of Ig light chain.

### Figure 11

Intraperitoneal administration of F991 (50 µg/animal) at 4 hours or 24 hours before hapten challenge of Ig light chain-sensitized mice completely inhibits induction of ear swelling after hapten challenge. Mice were i.v. sensitized with 2 µg TNP-specific Ig light chain at 30 min before ear challenge with TNP. Control mice were injected with PBS instead of Ig light chain.

### Figure 12

Subcutaneous administration of F991 (50 µg/animal) at 4 hours or 24 hours before hapten challenge of Ig light chain-sensitized mice completely inhibits induction of ear swelling after hapten challenge. Mice were i.v. sensitized with 2 µg TNP-specific Ig light chain at 30 min before ear challenge with TNP. Control mice were injected with PBS instead of Ig light chain.

### Figure 13

Topical application of F991 as an ointment on the ears results in dose-dependent inhibition of earswelling after hapten challenge of Ig light chain-sensitized animals. Application of F991 at 100 µg/g cream corresponds with a local dose of 20 µg per animal.

### Figure 14

Dorsal application (on back of mice) of F991 in ointment does not result in inhibition of earswelling after hapten challenge of Ig light chain-sensitized animals.

### Figure 15

F991 retains its activity when stored as ointment at room temperature or at 4 °C for more than 3 months.

### Figure 16

Topical treatment of DNFB-sensitized mice at 4 hours before hapten challenge results in complete inhibition of the development of contact sensitivity response as determined by earswelling at 2 and 24 hours after challenge.

### Figure 17

Induction of EAE in MOG/pertussis toxin treated mice is completely suppressed by daily administration of F991 (50 µg/animal i.p.).

## Claims

1. An isolated cell comprising an immunoglobulin-free-light-chain (Ig-LC) receptor capable of activating a signal transduction pathway in said cell upon binding of an Ig-LC to said receptor, wherein said signal transduction is independent of the presence of a functional common gamma-chain associated receptor on said cell.

2. An isolated cell according to claim 1, wherein said cell comprises a gamma chain-receptor deficient cell

3. An isolated cell according to claim 1 or claim 2, wherein said signal transduction pathway in said cell is activated upon binding of an Ig-LC to said receptor and subsequent cross-linking of receptor-bound Ig-LC.

4. An isolated cell according to any one of claim 1-3, wherein said cell comprises a mast cell.

5. An isolated cell according to any one of claims 1-4, wherein said receptor comprises an amino-acid sequence that is identical to the amino acid sequence of an endogenous gamma-chain-independent Ig-LC receptor encoded by the genome of said cell.

6. An isolated and/or recombinant Ig-LC receptor or a functional part, derivative and/or analogue thereof, capable of activating an Ig-LC dependent signal transduction pathway in a cell, wherein said activation is independent of the presence of a functional gamma-chain receptor on said cell.

7. A receptor according to claim 6, wherein said signal transduction pathway comprises an ion channel.

8. An isolated and/or recombinant nucleic acid encoding a receptor according to claims 6 or 7

9. Use of a cell according to any one of claims 1-5, or a receptor according to claims 6 or 7, or a nucleic acid according to claim 8 for selecting a compound capable of preventing binding of Ig-LC to said receptor.

10. A method for at least in part inhibiting Ig-LC induced signal transduction in a cell comprising providing said cell with a compound capable of at least in part inhibiting association of said Ig-LC with an Ig-LC receptor on said cell.

11. A method according to claim 10, wherein said receptor is a receptor for Ig-LC capable of activating an Ig-LC dependent signal transduction pathway in a cell, independent of the presence of a functional immunoglobulin on said cell.

12. A method according to claim 10 or claim 11, wherein said compound is capable of specifically binding to said receptor for Ig-LC.

13. A method according to claim 12, wherein said compound comprises Ig-LC selected for its capacity not to elicit signal transduction by the Ig-LC receptor.

14. A method according to any one of claims 10-13, wherein said compound is capable of specifically binding Ig-LC.

15. A method according to claim 14, wherein said compound comprises a peptide comprising an amino acid sequence (AHWSGHCCL).

16. A method for reducing a hypersensitivity response in an animal comprising providing said animal with a compound capable of at least in part inhibiting binding of an Ig-LC to a gamma chain independent receptor for Ig-LC.

17. A method for determining whether a compound is capable of at least in part inhibiting signal transduction of a gamma chain independent Ig-LC receptor comprising providing a gamma-chain-receptor deficient cell comprising said receptor with said compound and determining whether Ig-LC mediated signal transduction is at least in part inhibited in said cell.

18. A compound capable of at least in part inhibiting signal transduction of a gamma chain independent Ig-LC receptor obtainable by a method according to claim 17.

19. Use of a compound according to claim 18, for preparing a medicament for the treatment of (contact) dermatitis, asthma, psoriasis, inflammatory bowel disease, rheumatoid arthritis, Sjögren and systemic lupus erythematosus, and/or multiple sclerosis

20. A use according to claim 19, wherein said medicament is formulated and packaged for parenteral or oral administration

21. A method of treatment of an animal suffering from or at risk of suffering from (contact) dermatitis, asthma, psoriasis, inflammatory bowel disease, rheumatoid arthritis, Sjögren and systemic lupus erythematosus, and/or multiple sclerosis comprising administering to said animal a compound according to claim 18 with a carrier to a suitable recipient.
